# NEUE EUROPÄISCHE PATENTSCHRIFT

(11) **EP 1 457 174 B2**
(45) Veröffentlichungstag und Bekanntmachung des Hinweises auf die Entscheidung über den Einspruch: **31.12.2014**
(45) Hinweis auf die Patenterteilung: 22.04.2009
(21) Anmeldenummer: 04004289.7
(22) Anmeldetag: 26.02.2004
(51) Int. Cl.: A61F 2/68, A61F 2/64

(54) **Orthopädietechnisches Hilfsmittel mit einer Verriegelungsvorrichtung**
Orthopedic aid with locking device.
Moyen orthopédique avec dispositif de verrouillage.

(30) Priorität: 12.03.2003 DE 10311189
(43) Veröffentlichungstag der Anmeldung: 15.09.2004
(73) Patentinhaber: Otto Bock HealthCare GmbH, 37115 Duderstadt (DE)
(72) Erfinder: Lidolt, Klaus, 37115 Duderstadt (DE); Schilling, Matthias, 37345 Weissenborn-Lüderode (DE)
(74) Vertreter: Lins, Edgar

(56) Entgegenhaltungen:
- EP-A- 0 141 640
- EP-A- 0 205 785
- EP-B1- 0 359 760
- WO-A2-00/78263
- WO-A2-01/76515
- DE-A- 2 314 510
- DE-U1- 20 005 559
- US-A- 3 408 660
- US-A- 5 121 742
- US-A- 5 840 047
- US-B1- 6 517 503
- US-B1- 6 872 187
- 'Taking the Guesswork out of Walking' PROSTHETIC & ORTHOTIC UPDATE Nr. 38, 17 Dezember 2003, Seiten 1 - 4
- P.J. KYBERD ET AL.: 'A clinical experience with a hierarchically controlled myoelectric hand prosthesis with vibro-tactile feedback' PROSTHETICS AND ORTHOTICS INTERNATIONAL Bd. 17, 1993, Seiten 56 - 64
- P. HERBERTS ET AL.: 'Ideas on sensory feedback in hand prostheses' PROSTHETICS AND ORTHOTICS INTERNATIONAL Bd. 3, 1979, Seiten 157 - 162
- 'Grundkurs Technische Orthopädie', 2002, GEORG THIEME VERLAG Seiten 54 - 57
- Northern Orthopedics Update, No. 28, 2003
- The Hinnant Prosthetics Quarterly, Winter 2003-20C

## Beschreibung

Die Erfindung betrifft ein orthopädietechnisches Hilfsmittel für einen Patienten mit zwei relativ zueinander bewegbaren Teilen und einer Verriegelungsvorrichtung zum Verriegeln der beiden Teile in einer vorbestimmten relativen Position und zum Entriegeln der Teile zur Freigabe der Bewegung der Teile zueinander.

Orthopädietechnische Hilfsmittel dieser Art werden für zahlreiche Anwendungen eingesetzt, um am menschlichen Körper vorübergehend oder dauernd vorhandene Schwächen zu kompensieren und sonst nicht ausübbare Funktionen zu ermöglichen. Dies geschieht dadurch, dass das orthopädietechnische Hilfsmittel eine Stützfunktion ausübt, indem in einer bestimmten Stellung der Teile des Hilfsmittels diese Teile zueinander verriegelt werden, wobei die verriegelte Stellung der beiden Teile zueinander einer Gebrauchsstellung entspricht, in der der betreffende Patient die Stützfunktion durch das orthopädietechnische Hilfsmittel benötigt. Ein bevorzugter Anwendungsfall eines derartigen orthopädietechnischen Hilfsmittels ist die Ausbildung als Gelenkorthese, wobei beispielsweise die durch ein Gelenk miteinander verbundenen Teile der Gelenkorthese in gestreckter Stellung verriegelbar sind, um beipsielsweise die Funktion einer Extremität in der gestreckten und verriegelten Stellung der Gelenkorthese zu ermöglichen. Um in eine Ruhestellung zu gelangen, muss die Verriegelungsvorrichtung entriegelt werden. Dies geschieht beispielsweise bei bekannten Kniegelenkorthesen durch einen Bowdenzug, mit dem die Verriegelungsvorrichtung entriegelbar ist, sodass das Kniegelenk, beispielsweise für den Übergang in die sitzende Position des Patienten, gebeugt werden kann. Nach dem Aufstehen des Patienten muss die Kniegelenkorthese wieder in die gestreckte, verriegelte Stellung gelangen. Wird die Verriegelung nicht erreicht und dies von dem Patienten nicht bemerkt, besteht eine erhebliche Unfallgefahr, da beim Belasten der vermeintlich verriegelten Kniegelenkorthese diese einknicken kann und das Einknicken durch den Patienten aufgrund seiner Gelenkschwäche nicht kontrollierbar ist. Der Patient kann dabei unkontrolliert stürzen, Es bedarf daher einer besonderen Sorgfalt des Patienten, auf die tatsächlich erfolgte Verriegelung des Orthopädiemittels zu achten, indem beispielsweise ein für das Einrasten der Verriegelungsvorrichtung charakteristisches Geräusch abgewartet wird, bevor die Belastung des Hilfsmittels erfolgt.

Durch US 3,408,660 ist eine Prothese für einen Oberschenkelamputierten bekannt, bei der das Kniegelenk arretierbar ausgestaltet ist. Die Arretierung erfolgt dabei durch einen Rasthaken, der in seine Arretierungslage durch eine Feder gedrückt wird. Demgemäß wird bei jeder Streckung des Beins das Kniegelenk arretiert. In dem zur Prothese gehörenden künstlichen Fuß ist im Ballenbereich ein Pneumatikkissen untergebracht, das mit einem weiteren Pneumatikkissen unterhalb des Kniegelenks kommuniziert. Dieses zweite Pneumatikkissen betätigt bei seinem Aufpumpen einen Entarretierungsmechanismus für den Arretierungshebel. Da beim Abrollen des Fußes über den Ballen beim Gehzyklus das in der Sohle des künstlichen Fußes befindliche erste Luftkissen zusammengedrückt wird, erfolgt dabei ein Aufpumpen des zweiten Luftkissen, und damit eine Entarretierung des Kniegelenks, sodass beim Vorschwingen des Fußes in der Schwungphase des Gehzyklus das Kniegelenk einknicken kann. Durch den Schwung wird das Bein vor dem sich anschließenden Fersenauftritt wieder gestreckt und damit arretiert, sodass beim Fersenauftritt bis zum Überrollen auf den Ballenbereich des Fußes das Kniegelenk arretiert bleibt. Die beiden Luftkissen bilden mit ihrer Schlauchverbindung ein abgeschlossenes Luftvolumen. Aufgrund unvermeidbarer Undichtigkeiten wird sich die in dem Luftvolumen eingeschlossene Luftmenge verringern, sodass unter Umständen eine zuverlässige Entarretierungsfunktion nicht mehr gewährleistet ist. Die Stellung des von dem zweiten Luftkissen betätigten Mechanismus wird an der Vorderseite des künstlichen Unterschenkels durch einen mit dem Mechanismus verbundenen Zeiger und einer Skala angezeigt. Eine unterhalb der Anzeigevorrichtung befindliche Handpumpe ermöglicht eine Ergänzung des Luftvolumens, bis ein auf der Skala ablesbarer Sollbereich für die in dem Luftvolumen befindliche Luftmenge wieder erreicht ist. Die Anzeige ist für den Patienten mit der angelegten Prothese nicht ablesbar. Die Ablesung muss daher durch eine Hilfsperson bei angelegter Prothese oder bei abgenommener Prothese erfolgen.

EP 0 141 640 A1 offenbart ein Kniegelenk, bei dem das Oberschenkelteil und das Unterschenkelteil durch einen in eine Aufnahme des Unterschenkelteils einrastbaren Verriegelungsbolzen des Oberschenkelteils gegeneinander verriegelbar sind. Die Entriegelung ist über eine Steuerung durch einen Elektromagneten möglich. Das Herausziehen des Verriegelungsbolzens aus der Aufnahme des Unterschenkelteils wird mit einer Lichtschranke kontrolliert. Wenn der Bolzen herausgezogen ist, wird über eine Zeitsteuerung der Aktivierungsstrom für die Magnetspule abgestellt, um einen stromsparenden Betrieb zu ermöglichen, indem die Aktivierung der Magnetspule nur für eine kurze benötigte Zeit erfolgt. Ein am Oberschenkelteil angeordneter Endschalter erkennt durch eine auflaufende Kante des Unterschenkelteils die vollständig gestreckte Stellung der Beinprothese. Dieses Signal wird in der Steuereinheit als Vorbedingung für die Ansteuerung des Stroms zur Magnetspule angesehen.

Die vorliegende Erfindung geht von dem Problem aus, dass durch eine gewisse Nachlässigkeit des Benutzers des orthopädietechnischen Hilfsmittels die sorgfältige Vergewisserung des tatsächlichen Verriegelns der Verriegelungsvorrichtung unterbleibt und dadurch eine Unfallgefahr besteht. Der vorliegenden Erfindung liegt daher die Problemstellung zugrunde, eine Beeinträchtigung des Trägers des orthopädietechnischen Hilfsmittels durch eine versehentlich nicht vollständige Verriegelung der Teile des Hilfsmittels zueinander soweit wie möglich zu vermeiden.

Ausgehend von dieser Problemstellung ist erfindungsgemäß ein orthopädietechnisches Hilfsmittel durch die Merkmale des Patentanspruchs 1 gekennzeichnet.

Die erfindungsgemäße Signaleinrichtung generiert somit ein eigenes Signal, das den Verriegelungszustand (verriegelt oder entriegelt) für den Benutzer des orthopädietechnischen Hilfsmittels gut erkennbar anzeigt.

Dast orthopädietechnische Hilfsmittel weist wenigstens eine Detektionseinrichtung zur Erkennung des Verriegelungszustands (Verriegelung und/oder Entriegelung) der beiden Teile und zur Abgabe eines den Verriegelungszustand anzeigenden Signals auf. Bevorzugt ist dabei die Anzeige der Verriegelung, die somit als Quittungssignal für den Benutzer fungiert und ihm verdeutlicht, dass die angestrebte Verriegelung tatsächlich erfolgt ist, sodass das orthopädietechnische Hilfsmittel risikolos belastet werden kann.

Die Signaleinrichtung kann ferner zur Abgabe eines Signals bei der Entriegelung ausgelegt sein und als Warnsignal für den Fall einer versehentlichen Entriegelung der Verriegelungsvorrichtung dienen.

Das abgegebene Signal kann optisch, akustisch, haptisch und/oder mechanisch sein. Vorzugsweise werden die Signale in mehreren Formen ausgegeben, um ein Übersehen bzw. Überhören des Signals zu vermeiden. Neben der optischen und akustischen Wiedergabe des Signals kann daher eine mechanische Signalwiedergabe in Form von Vibrationen oder eine haptische Signalwiedergabe erfolgen, wenn beispielsweise für den Gebrauch des orthopädietechnischen Hilfsmittels zwangsläufig ein Griff, beispielsweise einer Gehhilfe, angefasst werden muss.

Die Detektionseinrichtung kann in Abhängigkeit vom Verriegelungszustand zur elektrischen Erzeugung des Signals ausgelegt sein. Dabei wird vorzugsweise auch der Verriegelungszustand selbst mit elektrischen Mitteln detektiert. Dies kann beispielsweise dadurch erfolgen, dass die Verriegelungsvorrichtung einen bewegbaren Verriegelungsstift aufweist, dessen Position durch die Detektionseinrichtung detektierbar ist.

Die Verriegelungsvorrichtung kann in einer bevorzugten Ausführungsform der Erfindung zur Entriegelung elektromechanisch betätigbar ausgebildet sein. Bei einer mit einem Verriegelungsstift arbeitenden Verriegelungsvorrichtung kann in diesem Fall der Verriegelungsstift zur Entriegelung in eine Magnetspule ziehbar angeordnet sein. Die Betätigung erfolgt somit nach Art eines Elektromagneten.

Vorzugsweise ist die Detektionseinrichtung zur elektrischen Abtastung der Position des Verriegelungsstifts ausgebildet. Diese Abtastung kann nach Art eines Potentiometers oder vorzugsweise induktiv erfolgen.

Durch die elektromechanische Entriegelung der Verriegelungsvorrichtung kann dafür gesorgt werden, dass die Entriegelung auf die elektromechanische Weise nur möglich ist, wenn die Teile des orthopädietechnischen Hilfsmittels gemäß der verriegelten Stellung belastet werden, sodass eine Entriegelung nicht möglich ist, wenn eine Kraft auf die Teile ausgeübt wird, die nicht der Position der Teile in der verriegelten Stellung entspricht. Konkret kann das orthopädietechnische Hilfsmittel als Gelenkorthese ausgebildet sein, bei der die Teile der Orthese in einer gestreckten Stellung verriegelbar sind, wobei eine elektromagnetische Betätigungseinrichtung mit einer schwachen Betätigungskraft von nicht mehr als 2 N vorgesehen ist, und die Gelenkorthese in der gestreckten Stellung ein geringes Spiel aufweist, durch das eine freie Beweglichkeit des Verriegelungsmechanismus in der zur gestreckten Stellung gehörenden Belastung der Gelenkorthese gegeben ist, während bei einer ein Drehmoment der Gelenkorthese ausübenden Belastung der Verriegelungsmechanismus aufgrund von Reibkräften nicht durch die Betätigungseinrichtung entriegelbar ist. Vorzugsweise ist die Betätigungskraft der elektromagnetischen Betätigungseinrichtung ≤ 1 N.

In einer weiter bevorzugten Ausführungsform erfolgt die Betätigung der Verriegelungsvorrichtung mittels einer drahtlosen Übertragung eines Betätigungssignals. Für den Fall einer Beingelenkorthese ist regelmäßig die Benutzung einer Gehhilfe in Form einer Stütze oder auch in Form eines Gehwagens erforderlich. In diesem Fall ist es außerordentlich vorteilhaft, wenn das Betätigungssignal für die drahtlose Übertragung des Befehlssignals an einem Griff der Gehhilfe auslösbar ist.

Umgekehrt kann auch das Signal der erfindungsgemäßen Signaleinrichtung auf die Gehhilfe drahtlos übertragbar sein, wenn die Gehhilfe vorzugsweise eine optische und/oder akustische Signalanzeigeeinrichtung aufweist und/oder ein Griff der Gehhilfe mit einem durch das Signal betätigbaren Vibrator versehen ist.

So ist es möglich, sowohl die Betätigung der Verriegelungsvorrichtung, insbesondere zum Entriegeln, mittels der Gehhilfe auszulösen und auch an der Gehhilfe das von der erfindungsgemäß vorhandenen Signaleinrichtung abgegebene Quittungssignal oder Warnsignal zu empfangen.

Die Erfindung soll im Folgenden anhand eines in der Zeichnung dargestellten Ausführungsbeispiels näher erläutert werden. Es zeigen:
- Figur 1: eine Seitenansicht einer Beinorthese gemäß einer Ausführungsform der Erfindung
- Figur 2: eine zu der Beinorthese gemäß Figur 1 gehörende Gehhilfe in Form einer Stütze
- Figur 3: eine Detaildarstellung eines Gelenks der Orthese gemäß Figur 1 im gesperrten Zustand in einer Seitenansicht
- Figur 4: das Gelenk gemäß Figur 3 in einer Ansicht von hinten
- Figur 5: die Ansicht gemäß Figur 3 für das Gelenk im entriegelten Zustand
- Figur 6: die Ansicht gemäß Figur 4 für das Gelenk im entriegelten Zustand
- Figur 7: eine Seitenansicht des Gelenks gemäß Figur 5 im gebeugten Zustand
- Figur 8: eine Seitenansicht eines Steuermoduls mit einer Entriegelungstaste
- Figur 9: eine Draufsicht auf das Steuermodul gemäß Figur 8
- Figur 10: ein Blockschaltbild für die elektrischen Teile der Beinorthese gemäß Figur 1, die mit einem Steuermodul gemäß den Figuren 8 und 9 ausgerüstet ist
- Figur 11: ein Steuermodul für die Beinorthese gemäß Figur 1, die drahtlos ansteuerbar ist
- Figur 12: eine Draufsicht auf das Steuermodul gemäß Figur 11

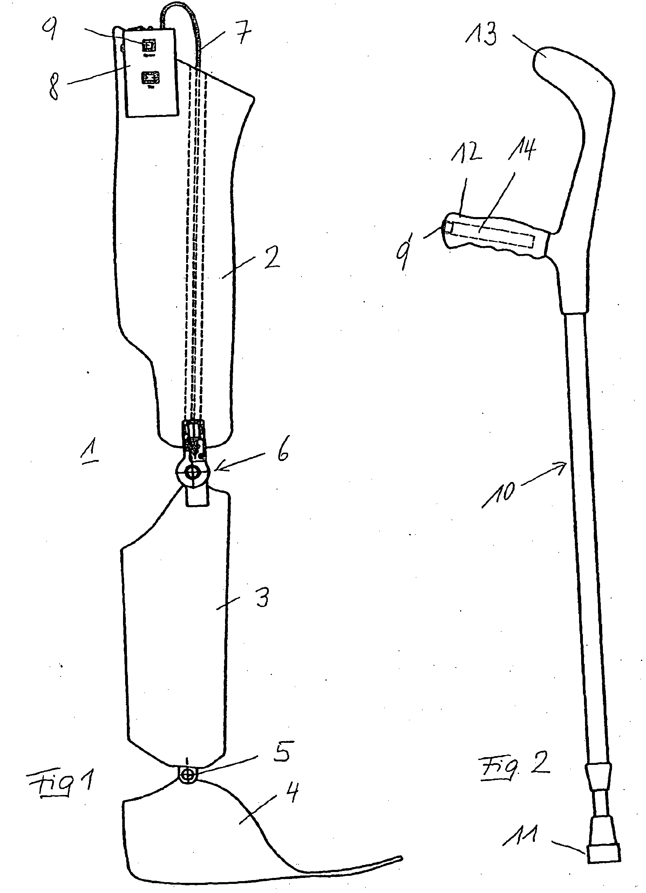

Die in Figur 1 dargestellte Beinorthese 1 weist eine Oberschenkelschale 2, eine Unterschenkelschale 3 und einen Fußaufnahmeschale 4 auf. Unterschenkelschale 3 und Fußaufnahmeschale 4 sind durch ein Drehgelenk 5 miteinander verbunden. Zwischen der Oberschenkelschale 2 und der Unterschenkelschale 3 ist ein verriegelbares Gelenk 6 angeordnet, das über ein Verbindungskabel 7 mit einem an der Oberseite der Oberschenkelschale 2 einhängbares Steuermodul 8 verbunden ist.

In Figur 1 nicht dargestellt sind in das Gelenk 6 einsteckbare flache Versteifungsstäbe, die mit der Obeschenkelschale 2 bzw. der Unterschenkelschale 3 verbindbar sind.

Das Gelenk 6 ist in der in Figur 1 dargestellten gestreckten Stellung verriegelbar und mittels einer Entriegelungstaste 9 des Steuermoduls 8 entriegelbar.

Figur 2 zeigt eine Gehhilfe 10 in Form einer stangenförmigen Stütze, die am unteren Ende ein zum Aufsetzen auf dem Boden vorgesehenes Gummiteil 11 und am oberen Ende einen Griff 12 und eine Unterarmstütze 13 aufweist. In den Griff 12 ist ein Auslöseschalter 9' integriert, der von der stirnseitigen Endfläche des Griffs 12 vorzugsweise mit dem Daumen betätigbar ist und der auf einen Sender 14 einwirkt, der daraufhin ein Betätigungssignal für das Steuermodul 8 aussenden kann. Das Steuermodul 8 ist in diesem Fall für einen Funkempfang eingerichtet.

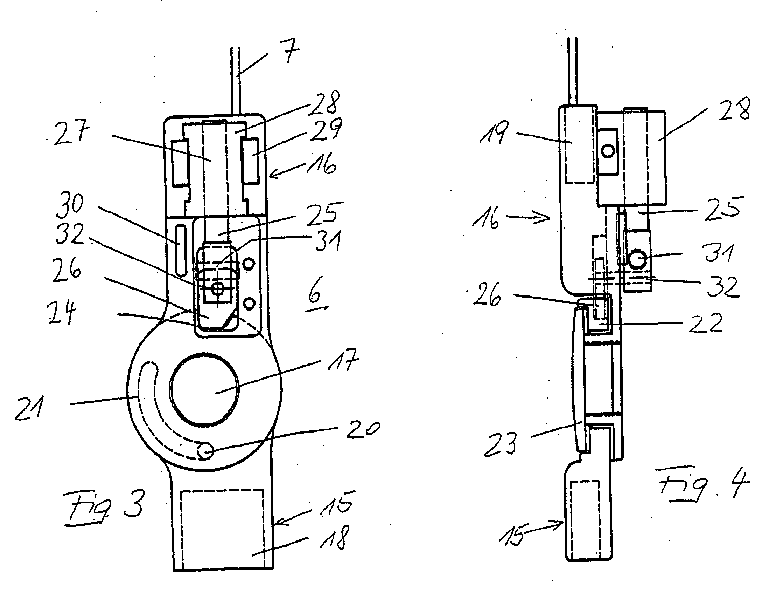

Der Aufbau des Gelenks 6 ist in den Figuren 3 bis 7 näher dargestellt. Das Gelenk 6 besteht aus zwei Gelenkteilen 15, 16 die über das Drehgelenk 17 drehbar miteinander verbunden sind.

Das Teil 15 ist als Gelenkunterteil mit einer nach unten offenen Aufnahmekammer 18 für einen flachen Versteifungsstab versehen, der mit der Unterschenkelschale 3 verbunden wird. In entsprechender Weise weist das Teil 16 eine nach oben offene Aufnahmekammer 19 zur Aufnahme eines Versteifungsstabs für die Oberschenkelschale 2 auf.

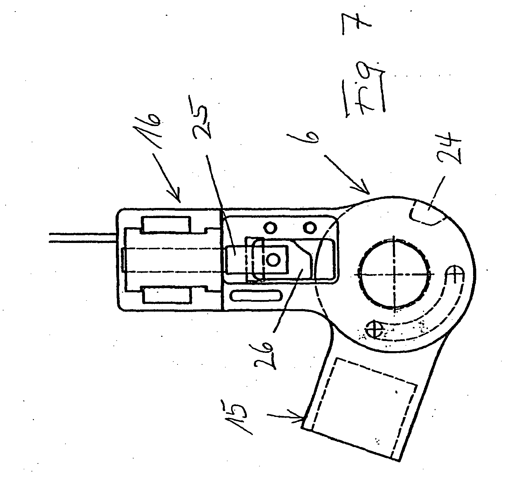

Das Gelenkunterteil 15 ist mit einem Führungsstift 20 versehen, der in einer etwa einen Viertelkreis ausbildenden Führungsnut 21 bewegbar ist und so Anschläge für die gestreckte Stellung gemäß Figur 3 und eine gebeugte Stellung gemäß Figur 7 des Gelenks 6 ausbildet.

Gelenkunterteil 15 und Gelenkoberteil 16 bilden im Bereich des Drehgelenks 17 beide kreisförmige, augenartige Endabschnitte 22, 23 aus, die ineinander zur Bildung des Drehgelenks 17 montiert sind. Der Endabschnitt 22 des Gelenkunterteils 15 ist mit einer radialen Ausnehmung 24 versehen, in die ein Verriegelungsstift 25 mit einem unteren, komplementär zur Ausnehmung 24 gefomten Ende 26 eingreift, um Gelenkunterteil 15 und Gelenkoberteil 16 in der in Figuren 3 und 4 dargestellten gestreckten Stellung miteinander zu verriegeln. Der Verriegelungsstift 25 geht an seinem oberen Ende in einen zylindrischen Kern 27 über, der im Innenraum einer elektrischen Spule 28 axial bewegbar ist. Die elektrische Spule 28 ist in einer Halterung 29 im Gelenkoberteil fixiert. Die Position des Verriegelungsstifts 25 ist mittels eines im Gelenkoberteil 16 neben dem Verriegelungsstift 25 angeordnete und sich parallel zu ihm erstreckenden Sensor 30 detektierbar. Mit dem Sensor 30 wirkt ein mit dem Verriegelungsstift 25 verbundender Permanentmagnet 31 zusammen, der sich quer zum Verriegelungsstift 27 erstreckt und dessen Magnetfeld durch den Sensor 30, der ein Hall-Sensor sein kann, detektierbar ist. In der in Figur 3 dargestellten verriegelten Stellung detektiert der Sensor 30 kein Magnetfeld des Permanentmagneten 31. Bewegt sich der Verriegelungsstift 25 nach oben, weil er aufgrund eines Stromflusses durch die Spule 28 in deren Innenraum gezogen wird, gelangt das Feld des Permanentmagneten 31 in den Bereich des Sensors 30, der somit den entriegelten Zustand detektiert. Sowohl der Strom für die Spule 28 als auch das Ausgangssignal des Sensors 30 werden über das Verbindungskabel 7 von bzw. zu dem Steuermodul 8 übertragen.

Figur 4 verdeutlicht, dass das Endstück 26 des Verriegelungsstifts 25 axial seitlich versetzt verfahrbar ist und mit dem Verriegelungsstift 25 über einen Verbindungssift 32 verbunden ist.

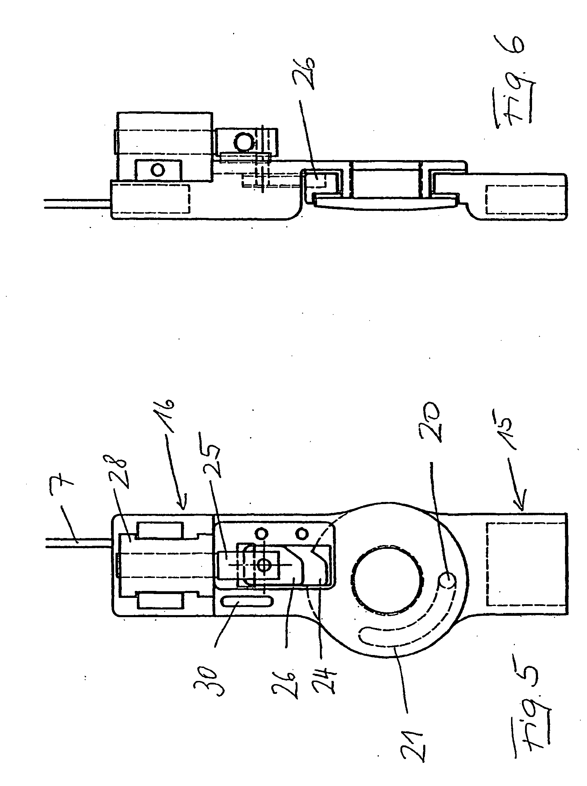

Die Figuren 5 und 6 zeigen das Gelenk 6 im entriegelten Zustand. Über das Verbindungskabel 7 wird die Spule 28 von Strom durchflossen und wirkt als Elektromagnet für den Verriegelungsstift 25, der in das Innere der Spule 28 - in der zeichnerischen Darstellung also nach oben - gezogen wird und das mit ihm verbundene Ende 26 aus der zugehörigen Ausnehmung 24 herauszieht, sodass das Gelenkunterteil 15 nunmehr gegenüber dem Gelenkoberteil 16 drehbar ist, und zwar im Rahmen der durch den Führungsstift 20 und die Führungsnut 21 vorgegebenen Führung 20, 21.

Figur 7 zeigt die gebeugte Endstellung des Gelenks 6, wie sie eingenommen wird, wenn sich der Benutzer setzt. Das Ende 26 des Verriegelungsstifts 25 gleitet dabei auf der zylinderischen Umfangsfläche des Endabschnitts 22 des Gelenkunterteils 15. Geht der Benutzer aus dem gebeugten Zustand gemäß Figur 7 in den gestreckten Zustand gemäß Figuren 3 bis 6 über, gleitet das untere Ende 26 des Verriegelungsstifts 15 unter dem Einfluss der Schwerkraft auf der Umfangsfläche des Endabschnitts 22, bis das untere Ende 26 in der vollständig gestreckten Stellung in die Ausnehmung 24 hineinfällt und die Verriegelung gemäß den Figuren 3 und 4 wirkt.

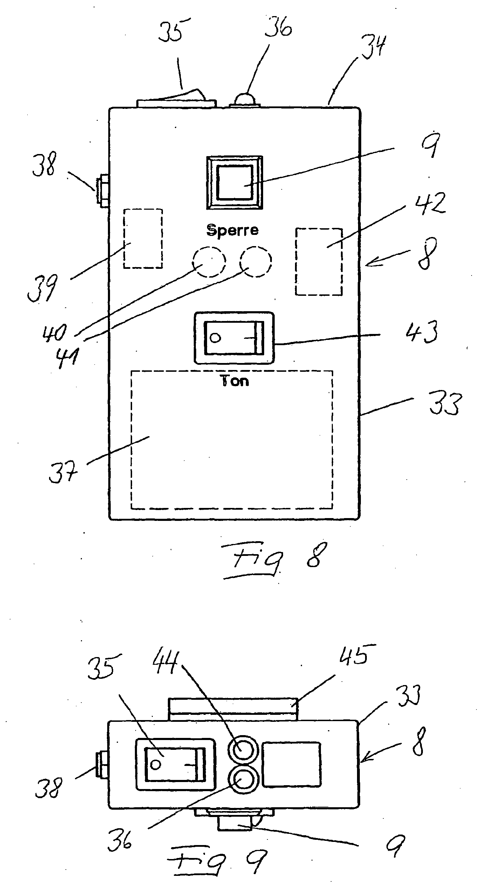

Das in den Figuren 8 und 9 dargestellte Steuermodul 8 weist die Taste 9 zum Entriegeln des Gelenks 6 an einer bequem erreichbaren Stelle auf. Das Steuermodul ist mit einem rechteckigen, flachen Gehäuse 33 versehen an dessen schmaler Oberseite 34 ein Hauptschalter 35 mit einer Warnleuchte 36 angeordnet ist. Das Gehäuse 33 beinhaltet an der Unterseite eine Batterie 37, die über eine an einer schmalen Seitenwand angebrachte Ladebuchse 38 wiederaufladbar ist. Das Steuermodul 8 beinhaltet ferner eine Kurzzeitsteuerung 39 und zwei Tongeneratoren 40, 41 sowie einen Vibrator 42. Über einen Schalter 43 können die Tongeneratoren 40, 41 abgeschaltet werden, um ein in bestimmten Situationen unerwünschtes akustisches Signal zu unterdrücken.

Figur 9 lässt erkennen, dass an der Oberseite 34 des Gehäuses 33 eine weitere Kontrollleuchte 44 vorgesehen ist, die den Ladezustand der Batterie 37 anzeigt. Ferner ist das Gehäuse 33 mit einem Klemmbügel 45 versehen, mit dem es an der Oberkante der Oberschenkelschale 2 klemmend befestigbar ist.

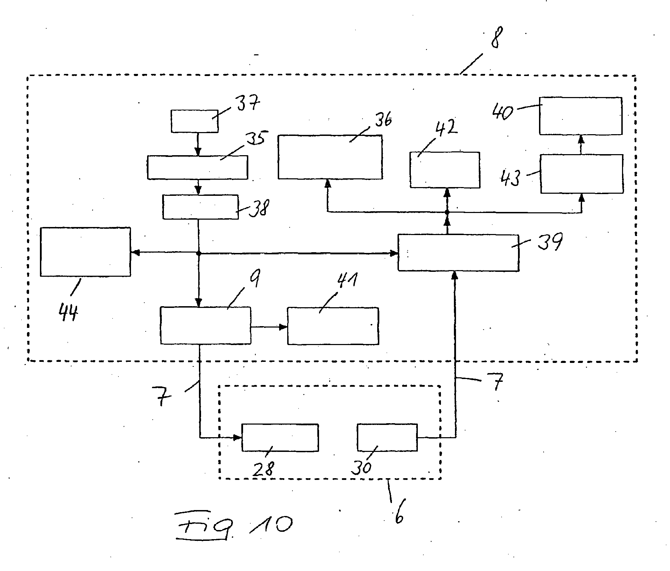

Das in Figur 10 dargestellte Blockschaltbild zeigt die funktionale Verschalung in dem Steuermodul 8 und die auf das Gelenk 6 über das Verbindungskabel 7 übertragenen Signale.

Die Batterie 37 ist über den Hauptschalter 35 mit der Ladebuchse 38 und der Taste 9 zum Entriegeln des Gelenks 6 verbunden. Wird die Taste 9 betätigt, wird damit der Tongenerator 41 angesteuert, der ein Warnton für die Entriegelung abgibt. Bei eingeschalteten Hauptschalter 35 wird der Ladezustand der Batterie 37 durch die Kontrollleuchte 44 angezeigt, indem die Kontrollleuchte 44 beispielsweise nicht aufleuchtet, wenn der Ladezustand der Batterie 37 ausreichend ist. Durch die Taste 9 wird ein Strom über das Verbindungskabel 7 in die Spule 28 im Gelenk 6 geleitet, wodurch die Entsperrung vorgenommen wird.

Detektiert der Sensor 30 des Gelenks 6, dass der Verriegelungsstift 25 wieder in die verriegelte Stellung gefallen ist, wird dieses Ausgangssignal des Sensors 30 über das Verbindungskabel 7 in das Steuermodul 8 übertragen und löst dort über die Kurzzeitsteuerung 39 Quittungssignale aus, nämlich durch ein über die Kurzzeitsteuerung 39 gesteuertes Aufleuchten der Kontrollleuchte 36, Betätigen des Vibrators 42 und Betätigen des Tongenerators 40, sofern dieser nicht über den Schalter 43 abgeschaltet worden ist.

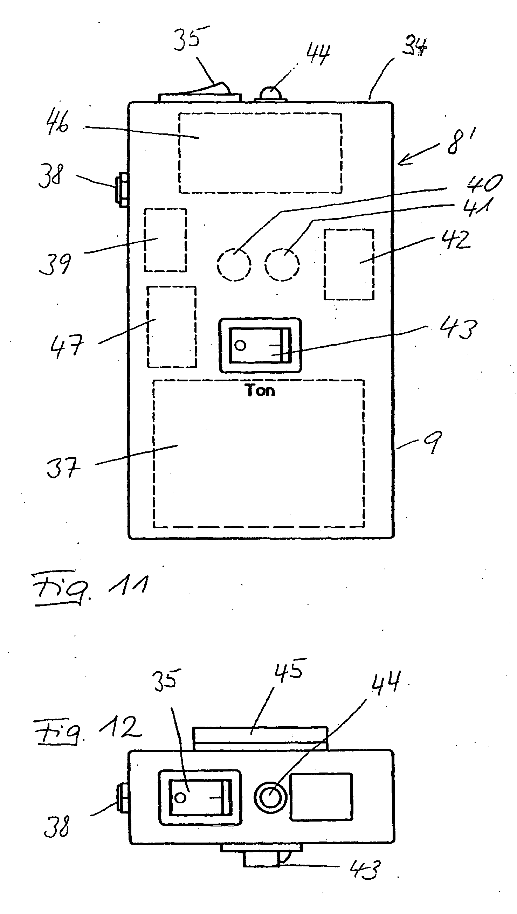

Die Figuren 11 und 12 zeigen das Steuermodul 8' in einer für einen Funkempfang von dem Sender 14 der Gehhilfe 10 ausgelöstes Betätigungssignal und ist daher zusätzlich mit einem Funkempfänger 46 und einem Schaltrelais 47 anstelle des Schalters 9 versehen. Selbstverständlich ist es auch möglich, zusätzlich zu dem Funkempfänger 46 und dem Schaltrelais 47 auch die Entriegelungsmöglichkeit über die Taste 9 in dem Steuermodul 8, 8' vorzusehen.

In der in den Figuren 11 und 12 dargestellten Ausführungsform ist wegen des Wegfalls des Schalters 9 auch die entsprechende Kontrollleuchte 36 entfallen, sodass sich an der Oberseite 34 des Gehäuses 9 nur noch die Kontrollleuchte 44 für den Ladezustand der Batterie 37 befindet.

Im Übrigen ist das Steuermodul 8' identisch zum Steuermodul 8 aufgebaut.

## Patentansprüche

1. Orthopädietechnisches Hilfsmittel für einen Patienten, mit zwei relativ zueinander bewegbaren Teilen (15, 16) und einer Verriegelungsvorrichtung zum Verriegeln der beiden Teile (15, 16) in einer vorbestimmten relativen Position und zum Entriegeln der Teile (15, 16) zur Freigabe der Bewegung der Teile (15, 16) zueinander, **dadurch gekennzeichnet, dass** wenigstens eine Detektionseinrichtung (30, 31) zur Erkennung des Verriegelungszustands der beiden Teile (15, 16) und wenigstens eine Signaleinrichtung (36, 40, 41, 42) vorgesehen ist, die ein für den Patienten gut erkennbares eigenes Anzeige- oder Warnsignal für den Verriegelungszustand der Verriegelungsvorrichtung abgibt.
wobei das Signal optisch, akustisch, haptisch und/oder mechanisch ist und
wobei die Detektionseinrichtung (30, 31) in Abhängigkeit vom Verriegelungszustand zur elektrischen Erzeugung des Signals ausgelegt ist.

2. Orthopädietechnisches Hilfsmittel nach Anspruch 1
**dadurch gekennzeichnet, dass** die Verriegelungsvorrichtung einen bewegbaren Verriegelungsstift (25) aufweist, dessen Position durch die Detektionseinrichtung (30, 31) detektierbar ist.

3. Orthopädietechnisches Hilfsmittel nach Anspruch 1 oder 2 **dadurch gekennzeichnet, dass** die Verriegelungsvorrichtung zur Entriegelung elektromechanisch betätigbar ausgebildet ist.

4. Orthopädietechnisches Hilfsmittel nach Anspruch 2 und 3, **dadurch gekennzeichnet, dass** zur Entriegelung der Verriegelungsstift (25) in eine Magnetspule (28) ziehbar angeordnet ist.

5. Orthopädietechnisches Hilfsmittel nach einem der Ansprüche 1 bis 4, **dadurch gekennzeichnet, dass** die Detektionseinrichtung (30, 31) zur elektrischen Abtastung der Position des Verriegelungsstifts ausgebildet ist.

6. Orthopädietechnisches Hilfsmittel nach einem der Ansprüche 1 bis 5, ausgebildet als eine Gelenkorthese, bei der die Teile (15, 16) des Gelenks (6) in einer gestreckten Stellung verriegelbar sind, **dadurch gekennzeichnet, dass** eine elektromagnetische Betätigungseinrichtung (28) mit einer schwachen Betätigungskraft von nicht mehr als 2 N ausgebildet ist und dass das Gelenk (6) in der gestreckten Stellung ein geringes Spiel aufweist, durch das eine freie Beweglichkeit des Verriegelungsmechanismus in der zur gestreckten Stellung gehörenden Belastung des Gelenks (6) gegeben ist, während bei einer ein Drehmoment des Gelenks (6) ausübenden Belastung der Verriegelungsmechanismus aufgrund von Reibkräften nicht durch die Betätigungseinrichtung (28) entriegelbar ist.

7. Orthopädietechnisches Hilfsmittel nach einem der Ansprüche 1 bis 6, **dadurch gekennzeichnet, dass** die Betätigung der Verriegelungsvorrichtung mittels einer drahtlosen Übertragung eines Betätigungssignals erfolgt.

8. Orthopädietechnisches Hilfsmittel nach Anspruch 7, **dadurch gekennzeichnet, dass** ein Betätigungssignal für die drahtlose Übertragung des Befehlssignals an einem Griff (12) einer Gehhilfe (10) auslösbar ist.

9. Orthopädietechnisches Hilfsmittel nach Anspruch 7 oder 8, **dadurch gekennzeichnet, dass** das Signal der Signaleinrichtung (36, 40, 41, 42) auf die Gehhilfe (10) drahtlos übertragbar ist.

10. Orthopädietechnisches Hilfsmittel nach Anspruch 9, **dadurch gekennzeichnet, dass** die Gehilfe (10) eine optische und/oder akustische Signalanzeigeeinrichtung aufweist.

11. Orthopädietechnisches Hilfsmittel nach Anspruch 9 oder 10, **dadurch gekennzeichnet, dass** ein Griff (12) der Gehhilfe (10) mit einem durch das Signal betätigbaren Vibrator versehen ist.

## Claims

1. An orthopedic aid for a patient with two parts (15, 16) which are movable relative to one another and with a locking device for locking the two parts (15, 16) in a predetermined relative position and for unlocking the parts (15, 16) in order to permit movement of the parts (15, 16) with respect to one another, **characterized in that** at least one detection arrangement (30, 31) is provided for detecting the locking state of the two parts (15, 16) and at least one signalling arrangement (36, 40, 41, 42) is provided which emits a particular indicator signal or warning signal for the locking state of the locking device which is readily identifiable for the patient in which the signal is visual, acoustic, tactile and/or mechanical, and in which the detection arrangement (30, 31) is designed to generate the signal electrically as a function of the locking state.

2. The orthopedic aid as claimed in claim 1, wherein the locking device has a movable locking pin (25) whose position can be detected by the detection arrangement (30, 31).

3. The orthopedic aid as claimed in claim 1 or 2, wherein the locking device is designed to be actuated electromechanically to permit unlocking.

4. The orthopedic aid as claimed in claims 2 and 3, wherein locking pin (25) is arranged such that it can be drawn into a magnet coil (28) to permit unlocking.

5. The orthopedic aid as claimed in one of claims 1 to 4, wherein the detection arrangement (30, 31) is designed for electrical sensing of the position of the locking pin.

6. The orthopedic aid as claimed in one of claims 1 to 5, designed as an orthotic joint in which the parts (15, 16) of the joint (6) can be locked in an extended position, wherein an electromagnetic actuating arrangement (28) with a low actuating force of not more than 2 N is provided, and wherein the joint (6) in the extended position has a slight play, allowing a freedom of movement of the locking mechanism in the loading of the joint (6) pertaining to the extended position, whereas, in the event of a load exerting a turning moment of the joint (6), the locking mechanism cannot be unlocked by the actuating arrangement (28) on account of frictional forces.

7. The orthopedic aid as claimed in one of claims 1 to 6, wherein the locking device is actuated by wireless transmission of an actuating signal.

8. The orthopedic aid as claimed in claim 7, wherein an actuating signal for wireless transmission of the command signal can be triggered on a handgrip (12) of a walking aid (10).

9. The orthopedic aid as claimed in claim 7 or 8, wherein the signal of the signaling arrangement (36, 40, 41, 42) can be sent by wireless transmission to the walking aid (10).

10. The orthopedic aid as claimed in claim 9, wherein the walking aid (10) has a visual and/or acoustic signal display arrangement.

11. The orthopedic aid as claimed in claim 9 or 10, wherein a handgrip (12) of the walking aid (10) is provided with a vibrator that can be actuated by the signal.

## Revendications

1. Moyen d'assistance orthopédique pour un patient, comprenant deux parties (15, 16) déplaçables l'une par rapport à l'autre et un dispositif de verrouillage pour verrouiller les deux parties (15, 16) dans une position relative prédéterminée et pour déverrouiller les parties (15, 16) pour libérer le mouvement des parties (15, 16) l'une par rapport à l'autre, **caractérisé en ce qu'**il est prévu au moins un dispositif de détection (30, 31) pour reconnaître l'état de verrouillage des deux parties (15, 16) et au moins un dispositif de signalisation (36, 40, 41, 42), qui envoie un signal d'avertissement ou d' indication propre bien reconnaissable pour le patient, pour l'état de verrouillage, ou lors du déverrouillage du dispositif de verrouillage que le signal est optique, acoustique, tactile et/ou mécanique, et que le dispositif de détection (30, 31) est conçu pour produire électriquement le signal en fonction de l'état de verrouillage.

2. Moyen d'assistance orthopédique selon la revendication 1, **caractérisé en ce que** le dispositif de verrouillage présente une goupille de verrouillage (25) déplaçable dont la position est détectable par le dispositif de détection (30, 31).

3. Moyen d'assistance orthopédique selon la revendication 1 ou 2, **caractérisé en ce que** le dispositif de verrouillage est actionnable par voie électromécanique pour le déverrouillage.

4. Moyen d'assistance orthopédique selon les revendications 2 et 3, **caractérisé en ce que** pour le déverrouillage, la goupille de verrouillage (25) est montée pour être rétractable dans une bobine magnétique (28).

5. Moyen d'assistance orthopédique selon l'une des revendications 1 à 4, **caractérisé en ce que** le dispositif de détection (30, 31) est conçu pour explorer électriquement la position de la goupille de verrouillage.

6. Moyen d'assistance orthopédique selon l'une des revendications 1 à 5, formé en tant qu'orthèse articulaire dans laquelle les parties (15, 16) de l'articulation (6) sont verrouillables dans la position d'extension, **caractérisé en ce qu'**un dispositif d'actionnement (28) électromagnétique est conçu avec une faible force d'actionnement ne dépassant pas 2 N, et **en ce que** dans la position d'extension, l'articulation (6) présente un faible jeu grâce auquel on obtient une libre mobilité du mécanisme de verrouillage sous la charge de l'articulation (6) correspondant à la position d'extension, tandis que pour une charge exerçant un moment de l'articulation (6), le mécanisme de verrouillage n'est pas déverrouillable par le dispositif d'actionnement (28) en raison de forces de friction.

7. Moyen d'assistance orthopédique selon l'une des revendications 1 à 6, **caractérisé en ce que** l'actionnement du dispositif de verrouillage s'effectue au moyen d'une transmission sans fil d'un signal d'actionnement.

8. Moyen d'assistance orthopédique selon la revendication 7, **caractérisé en ce qu'**un signal d'actionnement pour la transmission sans fil du signal de commande est déclenchable sur une poignée (12) d'un dispositif d'aide à la marche (10).

9. Moyen d'assistance orthopédique selon la revendication 7 ou 8, **caractérisé en ce que** le signal du dispositif de signalisation (36, 40, 41, 42) est transmissible sans fil au dispositif d'aide à la marche (10).

10. Moyen d'assistance orthopédique selon la revendication 9, **caractérisé en ce que** le dispositif d'aide à la marche (10) présente un dispositif d'indication de signal, optique et/ou acoustique.

11. Moyen d'assistance orthopédique selon la revendication 9 ou 10, **caractérisé en ce qu'**une poignée (12) du dispositif d'aide à la marche (10) est munie d'un vibreur actionnable par le signal.
